(19) Europäisches Patentamt
European Patent Office
Office européen des brevets

(11) EP 2 402 302 B1

(12) **EUROPEAN PATENT SPECIFICATION**

(45) Date of publication and mention of the grant of the patent:
**22.11.2017 Bulletin 2017/47**

(21) Application number: **10746125.3**

(22) Date of filing: **18.02.2010**

(51) Int Cl.:
*C07C 41/03* (2006.01) *B01J 27/053* (2006.01)
*C07C 43/13* (2006.01) *C07B 61/00* (2006.01)

(86) International application number:
**PCT/JP2010/052397**

(87) International publication number:
**WO 2010/098244 (02.09.2010 Gazette 2010/35)**

(54) **PROCESS FOR PRODUCING ETHER COMPOUND**

VERFAHREN ZUR ETHERERZEUGUNG

PROCÉDÉ DE PRODUCTION D'UN COMPOSÉ ÉTHER

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **24.02.2009 JP 2009041127**
**11.12.2009 JP 2009282125**

(43) Date of publication of application:
**04.01.2012 Bulletin 2012/01**

(73) Proprietor: **Kao Corporation**
**Chuo-Ku**
**Tokyo 103-8210 (JP)**

(72) Inventors:
- **MATSUMOTO, Tomotaka**
**Wakayama-shi**
**Wakayama 640-8580 (JP)**
- **NISHI, Takafumi**
**Wakayama-shi**
**Wakayama 640-8580 (JP)**
- **UNO, Mitsuru**
**Wakayama-shi**
**Wakayama 640-8580 (JP)**

(74) Representative: **Hoffmann Eitle**
**Patent- und Rechtsanwälte PartmbB**
**Arabellastraße 30**
**81925 München (DE)**

(56) References cited:

| | |
|---|---|
| **JP-A- S5 633 033** | **JP-A- H11 263 621** |
| **JP-A- H11 315 043** | **JP-A- 2001 179 105** |
| **JP-A- 2006 111 574** | **JP-A- 2006 199 560** |
| **JP-A- 2008 308 424** | **US-A- 4 727 199** |
| **US-A- 4 873 017** | **US-A- 5 025 094** |
| **US-A- 5 136 106** | **US-A1- 2006 270 883** |

- **BISWANATH OAS* ET AL: "SULFATED ZIRCONIA AS AN EFFICIENT RECYCLABLE HETEROGENEOUS CATALYST FOR SELECTIVE AMINOLYSIS OF EPOXIDES AND N-TOSYL AZIRIDINES UNDER SOLVENT-FREE CONDITION", INDIAN JOURNAL OF HETEROCYCLIC CHEMISTRY, INDIAN JOURNAL OF HETEROCYCLIC CHEMISTRY, IN, vol. 17, April 2008 (2008-04), pages 339-342, XP008163255, ISSN: 0971-1627**
- **DAS ET AL INDIAN JOURNAL OF HETEROCYCLIC CHEMISTRY vol. 17, 2008, pages 339 - 342, XP008163255**

EP 2 402 302 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

FIELD OF THE INVENTION

The present invention relates to a process for producing ether compounds from a hydroxyl group-containing compound and an epoxy compound in a simplified, efficient manner.

BACKGROUND OF THE INVENTION

Hitherto, β-halohydrin ethers have been produced by reacting a hydroxyl group-containing compound and an epoxy compound, for example, by reacting an alcohol and α-epihalohydrin in the presence of an acid catalyst. As the acid catalyst, there are known homogeneous Brϕnsted acids, homogeneous Lewis acids, heterogeneous solid acids or the like.

However, in the reaction using the homogeneous Brϕnsted acids such as sulfuric acid, there tend to occur the problems such as a low conversion rate of the α-epihalohydrin.

Also, in the reaction using the homogeneous Lewis acids such as those catalysts containing tin tetrachloride, boron trifluoride, an aluminum alkoxide, phenols or sulfonic acids (refer to JP 10-36307A), there tends to occur such a problem that α-epihalohydrin is further reacted with a halohydrin ether produced to thereby cause excessive addition of α-epihalohydrin thereto. Further, when the Lewis acid catalyst is in the form of a metal chloride, there tends to occur the problems such as deactivation of the catalyst owing to alcoholysis thereof, and reaction of the α-epihalohydrin with a chlorine group liberated therefrom. In addition, when using a high-activity Lewis acid such as boron trifluoride, there tends to occur the problems such as side reactions, e.g., polymerization of the α-epihalohydrin, etc.

Furthermore, in the reactions using the homogeneous Brϕnsted acid catalysts or Lewis acid catalysts, these catalysts are removed after completion of the reaction through steps including neutralization with alkalis and filtration. In these steps, if the alkalis are used in an excessive amount relative to the acids as the catalysts, the halohydrin ether produced tends to suffer from undesirable decomposition. Therefore, the neutralization step must be strictly controlled, so that the removal of the catalysts becomes extremely complicated.

On the other hand, as the methods using the heterogeneous solid acids, there are known the method using zirconium phosphoric acid salts (refer to JP 47-20606B), the method using a composite metal oxide (refer to JP 54-14910A), the method using an activated clay (refer to JP 7-133269A) or the like. These heterogeneous solid acid catalysts tend to be readily removed. However, since the heterogeneous solid acid catalysts generally have a low catalytic activity, there tends to occur such a problem that a large amount of the solid acid catalyst or a large amount of the alcohols must be used in the reaction to produce β-halohydrin ether with a high yield, and further the reaction time is prolonged.

USP 4,727, 199, USP 4,873,017 and USP 5,025,094 disclose the method in which an epoxy compound and an active hydrogen-containing compound are subjected to alkoxylation reaction in the presence of a heterogeneous catalyst formed of an anion-bonded metal oxide such as a non-crystalline sulfated zirconia, as well as a composition obtained by the method.

US 4,727,199 A relates to the alkoxylation of active-hydrogen compounds, such as primary and secondary alcohols or diols using solid anion-bound metal oxide catalysts, such as, zirconium oxysulfate catalyst. The catalysts are prepared by treating hydrous zicronium oxide with solutions of sulfate phosphate, nitrate or tetrafluoroborate and calcining in air at 300 °C to 950 °C. The amorphous catalysts are said to afford narrow molecular weight products. Reaction temperatures of 50 °C to 140 °C. are employed for alkoxylation.

SUMMARY OF THE INVENTION

The present invention relates to a process for producing an ether compound which includes the step of reacting a hydroxyl group-containing compound with an epoxy compound in the presence of an oxide of a metal of Group 4 of the Periodic Table on which a sulfate ion is supported, wherein

the hydroxyl group-containing compound is represented by the following general formula (1):

$$R^1\text{-}(OA^1)_n\text{-}(OA^2)_m\text{-}OH \qquad (1)$$

wherein $R^1$ is a hydrocarbon group having 1 to 36 carbon atoms; $A^1$ is an alkanediyl group having 2 to 4 carbon atoms; $A^2$ is a hydroxyl group-containing alkanediyl group having 2 to 4 carbon atoms; and n and m represent average polymerization degrees of the $OA^1$ group and the $OA^2$ group, respectively, in which n is a number of from 0 to 20 and m is a number of from 0 to 2.

the epoxy compound is an α-epihalohydrin and/or a 1,2-epoxy compound represented by the following general formula (2):

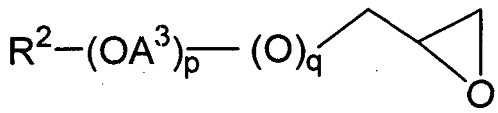 (2)

wherein $R^2$ is a hydrocarbon group having 1 to 36 carbon atoms; $A^3$ is an alkanediyl group having 2 to 4 carbon atoms; p is a number of from 0 to 20; and q is a number of 0 or 1, and
the metal of Group 4 of the Periodic Table is zirconium,
characterized in that
the sum of the diffraction intensities $I_{total}$ = ($I_{28.3}$+ $I_{30.3}$ + $I_{31.5}$) of the metal oxide is 2000 cps (counts per second) or larger as measured by subjecting the metal oxide to powder X-ray diffraction analysis,
wherein $I_{28.3}$ represents the diffraction intensity of the (-111) monoclinic lattice plane of the zirconia at a diffraction angle 2θ = 28.3°, $I_{30.3}$ represents the diffraction intensity of the (111) tetragonal lattice plane of the zirconia at a diffraction angle 2θ = 30.3°, and $I_{31.5}$ represents the diffraction intensity of the (111) monoclinic lattice plane of the zirconia at a diffraction angle 2θ = 31.5°,
wherein the value of $I_{total}$ is obtained under the measuring conditions and procedures described in the Examples below.

## BRIEF DESCRIPTION OF THE DRAWING

FIG. 1 shows an X-ray diffraction spectrum of sulfated zirconium oxide used in Examples 1 and 2, and an X-ray diffraction spectrum of sulfated zirconium oxide used in Comparative Examples 5 and 6.

## DETAILED DESCRIPTION OF THE INVENTION

The present invention relates to a process for producing an ether compound from a hydroxyl group-containing compound and an epoxy compound in a simplified, efficient manner.

The present inventors have found that when reacting the hydroxyl group-containing compound with the epoxy compound in the presence of a crystalline oxide of zirconium on which a sulfate ion is supported, the aimed ether compound can be obtained for a short period of time with an extremely high yield, and after completion of the reaction, the catalyst can be readily removed from the resulting reaction mixture.

That is, the present invention is directed to a process for producing an ether compound which includes the step of reacting a hydroxyl group-containing compound with an epoxy compound in the presence of an oxide of a metal of Group 4 of the Periodic Table on which a sulfate ion is supported, wherein the sum of the diffraction intensities $I_{total}$ = ($I_{28.3}$ + $I_{30.3}$ + $I_{31.5}$) of the metal oxide is 2000 cps (counts per second) or larger as measured by subjecting the metal oxide to powder X-ray diffraction analysis,
wherein $I_{28.3}$ represents the diffraction intensity of the (-111) monoclinic lattice plane of the zirconia at a diffraction angle 20 = 28.3°, $I_{30.3}$ represents the diffraction intensity of the (111) tetragonal lattice plane of the zirconia at a diffraction angle 2θ = 30.3°, and $I_{31.5}$ represents the diffraction intensity of the (111) monoclinic lattice plane of the zirconia at a diffraction angle 2θ = 31.5°,
the hydroxyl group-containing compound is represented by the following general formula (1):

$$R^1\text{-}(OA^1)_n\text{-}(OA^2)_m\text{-}OH \qquad (1)$$

wherein $R^1$ is a hydrocarbon group having 1 to 36 carbon atoms; $A^1$ is an alkanediyl group having 2 to 4 carbon atoms; $A^2$ is a hydroxyl group-containing alkanediyl group having 2 to 4 carbon atoms; and n and m represent average polymerization degrees of the $OA^1$ group and the $OA^2$ group, respectively, in which n is a number of from 0 to 20 and m is a number of from 0 to 2,
the epoxy compound is an α-epihalohydrin and/or a 1,2-epoxy compound represented by the following general formula (2):

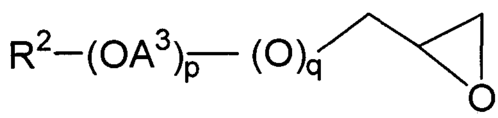 (2)

wherein $R^2$ is a hydrocarbon group having 1 to 36 carbon atoms; $A^3$ is an alkanediyl group having 2 to 4 carbon atoms; p is a number of from 0 to 20; and q is a number of 0 or 1, and
the metal of Group 4 of the Periodic Table is zirconium.

(Hydroxyl Group-Containing Compound)

The hydroxyl group-containing compound used in the present invention is a compound represented by the following general formula (1):

$$R^1\text{-}(OA^1)_n\text{-}(OA^2)_m\text{-}OH \qquad (1)$$

wherein $R^1$ is a hydrocarbon group having 1 to 36 carbon atoms; $A^1$ is an alkanediyl group having 2 to 4 carbon atoms; $A^2$ is a hydroxyl group-containing alkanediyl group having 2 to 4 carbon atoms; and n and m represent average polymerization degrees of the $OA^1$ group and the $OA^2$ group, respectively, in which n is a number of from 0 to 20 and m is a number of from 0 to 2.

In the general formula (1), from the viewpoint of good properties of the ether compound as the aimed product, $R^1$ is (i) a linear, branched or cyclic alkyl group preferably having 2 to 22 carbon atoms, more preferably 4 to 18 carbon atoms and still more preferably 6 to 12 carbon atoms; (ii) a linear, branched or cyclic alkenyl group preferably having 2 to 36 carbon atoms, more preferably 5 to 24 carbon atoms and still more preferably 8 to 18 carbon atoms; or (iii) a substituted or unsubstituted aromatic group. In the case where $R^1$ is a branched group, the number and positions of branched chains thereof are not particularly limited.

In addition, examples of the substituent groups which may be bonded to $R^1$ include halogen atoms such as a fluorine atom and a chlorine atom, a hydroxyl group and an alkoxy group.

Specific examples of the alkyl groups as $R^1$ include methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, isopropyl, 2-ethylhexyl, 3,5-dimethylhexyl, 3,5,5-trimethylhexyl, cyclopentyl, cyclohexyl and cyclooctyl groups.

Specific examples of the alkenyl groups as $R^1$ include allyl, oleyl, linoleyl, cyclopentenyl, cyclohexenyl and cyclooctenyl groups.

Specific examples of the substituted or unsubstituted aromatic groups as $R^1$ include phenyl, phenyl methyl, 2-methyl phenyl, 3-methyl phenyl, 4-methyl phenyl and naphthyl groups.

In the general formula (1), $A^1$ is a linear or branched alkanediyl group having 2 to 4 carbon atoms, preferably 2 to 3 carbon atoms and more preferably 2 carbon atoms from the viewpoints of good properties of the resulting ether compound as the aimed product. Specific example of the alkanediyl group as $A^1$ include an ethylene group, a trimethylene group and a propane-1,2-diyl group. Among these alkanediyl groups, preferred is an ethylene group.

Also, in the general formula (1), $A^2$ is a hydroxyl group-containing linear or branched alkanediyl group having 2 to 4 carbon atoms, preferably 2 to 3 carbon atoms and more preferably 3 carbon atoms from the viewpoints of good properties of the resulting ether compound as the aimed product. Specific example of the hydroxyl group-containing alkanediyl group as $A^2$ include a hydroxy-propane-1,2-diyl group and the like.

In addition, in the general formula (1), from the viewpoint of good properties of the resulting ether compound as the aimed product, n is a number of from 0 to 20, preferably from 0 to 8, more preferably from 0 to 3 and still more preferably 0, whereas m is a number of from 0 to 2, preferably 0 or 1 and more preferably 0.

The plural $OA^1$ groups and the plural $OA^2$ groups being present in a molecule of the hydroxyl group-containing compound may be respectively the same or different from each other.

Suitable examples of the hydroxyl group-containing compound represented by the general formula (1) include (a) alcohols, (b) alkylene glycol monoalkyl ethers and (c) glyceryl ethers.

Specific examples of the alcohols (a) include methanol, ethanol, propanol, butanol, pentanol, hexanol, heptanol, octanol, nonanol, decanol, undecanol, dodecanol, tetradecanol, hexadecanol, heptadecanol, octadecanol, nonadecanol, eicosanol, isopropanol, 2-ethyl hexanol, 3,5-dimethyl hexanol, isodecanol, isododecanol, allyl alcohol, oleyl alcohol, linolenyl alcohol, cyclopentanol, cyclohexanol, cyclooctanol, phenyl methanol, phenol and naphthol. Among these alcohols, preferred are one or more alcohols selected from the group consisting of pentanol, octanol, decanol, dodecanol (lauryl alcohol), tetradecanol, hexadecanol and 2-ethyl hexanol.

Specific examples of the alkylene glycol monoalkyl ethers (b) include ethylene glycol monomethyl ether, ethylene glycol monohexyl ether, ethylene glycol monostearyl ether, propylene glycol monomethyl ether, propylene glycol monohexyl ether, propylene glycol monooctyl ether, propylene glycol monododecyl ether, polyethylene glycol monododecyl ether, polyethylene glycol monomyristyl ether, polyethylene glycol monopalmityl ether and polypropylene glycol monododecyl ether.

Specific examples of the glyceryl ethers (c) include ethyl glyceryl ether, hexyl glyceryl ether, 2-ethylhexyl glyceryl ether, octyl glyceryl ether, decyl glyceryl ether, lauryl glyceryl ether, stearyl glyceryl ether, isostearyl glyceryl ether,

diglycerol monododecyl ether and ethylene glycol monododecyl monoglycerol ether.

These hydroxyl group-containing compounds may be used alone or in combination of any two or more thereof.

The hydroxyl group-containing compound is preferably used in an amount of from 0.8 to 10.0 mol per 1 mol of the epoxy compound. From the viewpoints of a high reactivity of the etherification and a high productivity of the aimed ether compound, the amount of the hydroxyl group-containing compound used is more preferably from 1.0 to 3.0 mol, still more preferably from 1.0 to 2.0 mol, further still more preferably from 1.1 to 1.8 mol and especially preferably from 1.1 to 1.3 mol per 1 mol of the epoxy compound.

<Epoxy Compound>

The epoxy compound used in the present invention is an α-epihalohydrins such as α-epichlorohydrin, α-epibromohydrin and α-epiiodohydrin, and/or a 1,2-epoxy compounds represented by the following general formula (2):

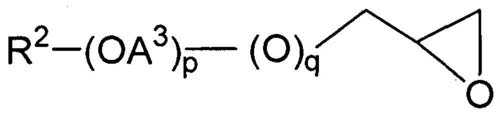
(2)

wherein $R^2$ is a hydrocarbon group having 1 to 36 carbon atoms; $A^3$ is an alkanediyl group having 2 to 4 carbon atoms; p is a number of from 0 to 20; and q is a number of 0 or 1.

Specific examples and suitable examples of the hydrocarbon group having 1 to 36 carbon atoms which is represented by $R^2$ in the general formula (2) are the same as the specific examples and suitable examples of the hydrocarbon group having 1 to 36 carbon atoms which is represented by $R^1$ in the general formula (1).

In addition, specific examples and suitable examples of the alkanediyl group having 2 to 4 carbon atoms which is represented by $A^3$ in the general formula (2) are the same as the specific examples and suitable examples of the alkanediyl group having 2 to 4 carbon atoms which is represented by $A^1$ in the general formula (1).

In the general formula (2), p is a number of from 0 to 20. From the viewpoint of good properties of the resulting ether compound, p is preferably a number of from 0 to 8, more preferably from 0 to 3 and still more preferably 0, whereas q is a number of 0 or 1, and preferably 1.

Among the 1,2-epoxy compounds represented by the general formula (2), preferred are alkyl glycidyl ethers in which $R^2$ is an alkyl group having 1 to 36 carbon atoms, preferably 5 to 18 carbon atoms and especially preferably 6 to 12 carbon atoms.

Specific examples of the epoxy compound suitably used in the present invention include one or more compounds selected from the group consisting of epichlorohydrin, 2-ethylhexyl glycidyl ether and dodecyl glycidyl ether.

The method of adding the epoxy compound is not particularly limited. For example, various addition methods such as collective (one-time) addition and divided (intermittent) addition, and continuous dropping or divided (intermittent) dropping may be used in the present invention. The reaction between the hydroxyl group-containing compound and the epoxy compound is an exothermic reaction. Therefore, from the viewpoint of well controlling the reaction, the epoxy compound is preferably added to the hydroxyl group-containing compound while stirring by either the divided (intermittent) addition method or the divided (intermittent) dropping method and more preferably by the divided (intermittent) dropping method.

<Crystalline Oxide of Metal of Group 4 of the Periodic Table on which Sulfate Ion is Supported>

In the present invention, there is used an oxide of zirconium as an oxide of a metal of Group 4 of the Periodic Table on which a sulfate ion is supported, which satisfies the condition that the sum of the diffraction intensities $I_{total} = (I_{28.3} + I_{30.3} + I_{31.5})$ of a diffraction intensity of a (111) crystal lattice plane and a diffraction intensity of a (-111) crystal lattice place of the metal oxide (hereinafter occasionally referred to merely as "$I_{total}$") is 2000 cps (counts per second) or larger as measured by subjecting the metal oxide to powder X-ray diffraction analysis (hereinafter occasionally referred to merely as a "crystalline sulfated Group 4 metal oxide").

$I_{total}$ of zirconia as the Group 4 metal oxide is represented by the following calculation formula (1):

$$I_{total} = (I_{28.3} + I_{30.3} + I_{31.5}) \qquad (1)$$

wherein $I_{28.3}$ represents the diffraction intensity of a (-111) monoclinic lattice plane of the zirconia (at a diffraction angle 2θ = 28.3°), $I_{30.3}$ represents the diffraction intensity of a (111) tetragonal lattice plane of the zirconia (at a diffraction

angle 2θ = 30.3°), and $I_{31.5}$ represents the diffraction intensity of a (111) monoclinic lattice plane of the zirconia (at a diffraction angle 2θ = 31.5°) as measured by a powder X-ray diffraction analysis.

The value of $I_{total}$ is calculated from a peak intensity observed in a powder-X-ray diffraction spectrum as measured under the conditions described in Examples below. When the value of $I_{total}$ is 2000 cps (counts per second) or larger, the metal oxide is defined as being crystalline.

The value of $I_{total}$ is preferably 2500 cps (counts per second) or larger, more preferably 3000 cps (counts per second) or larger and still more preferably from 3200 to 6000 cps (counts per second) from the viewpoint of a good catalytic activity.

The Group 4 metal contained in the crystalline sulfated Group 4 metal oxide is zirconium. Sulfate ion-supporting crystalline zirconium oxide (hereinafter referred to merely as a "crystalline sulfated zirconia") is preferred from the viewpoints of a good reactivity, etc.

The crystalline sulfated zirconia is not a compound having a stoichiometric composition such as zirconia sulfate ($Zr(SO_4)_2$), but has been reported to be a compound usually having an atomic ratio of S to Zr of less than 1 (S/Zr < 1) in which $SO_4$ is coordinated onto zirconia (refer to "PETEROTECH", Vol. 19, No. 9, p. 733 (1996)).

The content of the sulfate ion ($SO_4^{2-}$) in the crystalline sulfated zirconia, is preferably from 0.1 to 30% by mass, more preferably from 0.1 to 20% by mass, still more preferably from 1 to 20% by mass, further still more preferably from 1.5 to 15% by mass and especially preferably from 2 to 12% by mass from the viewpoint of a high reactivity and a good production stability.

The method of preparing the crystalline sulfated Group 4 metal oxide used in the present invention is not particularly limited as long as the resulting Group 4 metal oxide has $I_{total}$ which lies within the specific range as defined by the present invention. The crystalline sulfated Group 4 metal oxide may be prepared by conventionally known methods such as a liquid phase method and a solid phase method. For example, there may be mentioned the following preparation methods (A) and (B).

[Preparation Method A]

At least one basic compound selected from the group consisting of aqueous ammonia, sodium hydroxide and potassium hydroxide is added to and reacted with a basic metal sulfate compound (compound of a metal of Group 4 of the Periodic Table) such as basic zirconium sulfate to obtain a sulfuric acid-containing hydroxide (hydroxide of a metal of Group 4 of the Periodic Table) such as sulfuric acid-containing zirconium hydroxide. The thus obtained sulfuric acid-containing hydroxide may be recovered by known methods such as a co-precipitation method in which the obtained mixture is subjected to filtration, water-washing and then to solid-liquid separation. The thus recovered sulfuric acid-containing hydroxide may be dried, if required.

Next, the resulting sulfuric acid-containing hydroxide (hydroxide of a metal of Group 4 of the Periodic Table) is calcined to thereby obtain the crystalline sulfated Group 4 metal oxide.

[Preparation Method B]

A hydroxide or oxide of a metal of Group 4 of the Periodic Table selected from the group consisting of zirconium hydroxide, hydrous zirconium oxide and zirconium oxide is impregnated with a sulfuric acid compound selected from the group consisting of sulfuric acid, ammonium sulfate and a sulfate ion-containing compound.

Meanwhile, the hydrous zirconium oxide means zirconium hydroxide heat-treated below a temperature at which the zirconium hydroxide is transformed into zirconium oxide. The hydroxide or oxide of a metal of Group 4 of the Periodic Table selected from the group consisting of zirconium hydroxide, hydrous zirconium oxide and zirconium oxide as a raw material may have either a crystalline structure, a non-crystalline structure or a mixture of both the structures.

The product obtained after the impregnation treatment is calcined to obtain the crystalline sulfated Group 4 metal oxide.

The calcination temperature used for preparing the crystalline sulfated Group 4 metal oxide is preferably from 550 to 750°C, more preferably from 570 to 700°C and still more preferably from 575 to 650°C from the viewpoints of a high reactivity, a good production stability and a high activity upon recycling. The calcination time may be appropriately determined according to the calcination temperature, etc., and is preferably from 10 min to 24 h, more preferably from 0.5 to 10 h and still more preferably from 1 to 6 h which is the elapsed time as measured after the time at which the aimed calcination temperature is reached, from the viewpoints of a high reactivity, a good production stability and a high activity upon recycling. The calcination atmosphere may be either atmospheric air or an oxidative atmosphere.

The crystalline sulfated Group 4 metal oxide obtained by carrying out the calcination under the above conditions exhibits a high reactivity as a catalyst for production of the ether compound according to the present invention, and can maintain a high reactivity without any regeneration treatment, etc., even when the catalyst is repeatedly used.

Among these methods for preparation of the catalyst, preferred is the preparation method (B) in which the

hydroxide or oxide of a metal of Group 4 of the Periodic Table is used as a raw material.

The crystalline sulfated Group 4 metal oxide may contain a hydroxide thereof unless the aimed effects of the present invention are adversely affected. From the viewpoints of improvement in catalytic activity, etc., the crystalline sulfated Group 4 metal oxide may further contain and support thereon a metal of Group 10 or 11 of the Periodic Table such as palladium and platinum.

The configuration of the crystalline sulfated Group 4 metal oxide upon use is not particularly limited. The crystalline sulfated Group 4 metal oxide may be used in the form of a powder, may be molded into a pellet shape, a spherical shape, an ellipsoidal shape or a hollow cylindrical shape, or may be wash-coated on a refractory honeycomb carrier.

In the preferred embodiment, the crystalline sulfated Group 4 metal oxide may be dispersed in a reaction mixture or may be allowed to coexist therein as a so-called fixed bed catalyst. When dispersed in the reaction mixture, the crystalline sulfated Group 4 metal oxide may be readily separated or removed therefrom after completion of the reaction by a physical method such as filtration and centrifugal separation.

The above crystalline sulfated Group 4 metal oxides may be used alone or in combination of any two or more thereof.

The crystalline sulfated Group 4 metal oxide is preferably used in an amount of from 0.1 to 20% by mass, more preferably from 0.5 to 10% by mass and still more preferably from 2 to 10% by mass on the basis of the epoxy compound from the viewpoints of a high reactivity and a facilitated removal procedure after completion of the reaction.

Meanwhile, from the viewpoint of a good industrial convenience, the reaction is preferably carried out in a solvent-free condition. However, when the reaction system is in a highly viscous state or a non-uniform state owing to the kind or amount of the hydroxyl group-containing compound or epoxy compound added, the reaction may be conducted in the presence of an adequate amount of the following suitable solvent.

<Solvent>

Examples of the solvent include amphipatic solvents such as tetrahydrofuran, dioxane and ethylene glycol dimethyl ether; hydrocarbon-based solvents, e.g., aliphatic hydrocarbons such as hexane, heptane, cyclohexane, methyl cyclohexane, isooctane and hydrogenated triisobutylene, and aromatic hydrocarbons such as benzene, toluene, xylene and ethyl benzene; and silicone-based solvents such as octamethyl cyclotetrasiloxane and decamethyl cyclopentasiloxane.

These solvents may be used alone or in combination of any two or more thereof. Meanwhile, these solvents are preferably subjected to dehydration or deaeration upon use.

The solvent is preferably used in an amount of from 0 to 100 times by mass and more preferably from 0 to 10 times by mass on the basis of a total mass of the hydroxyl group-containing compound, the epoxy compound and the sulfated Group 4 metal oxide from the viewpoints of facilitated removal of the solvent, a high reactivity and a high productivity. Especially preferably, no solvent is used in the reaction.

<Reaction Conditions>

The reaction temperature used in the process of the present invention is preferably from 10 to 250°C, more preferably from 10 to 150°C, still more preferably from 60 to 130°C and especially preferably from 80 to 110°C from the viewpoints of reaction time, reaction efficiency, yield, selectivity and quality of the resulting ether compound.

The reaction pressure is not particularly limited, and the reaction may be carried out under applied pressures, if required.

The reaction method usable in the process of the present invention may be either a batch method, a semi-batch method or a continuous method, etc.

<Purification>

After completion of the reaction, the crystalline sulfated Group 4 metal oxide used therein may be readily removed from the reaction solution by a suitable method such as filtration, centrifugal separation and decantation. The thus obtained reaction solution may be then subjected to washing treatment, if required, thereby obtaining the ether compound as the aimed product.

If required, the resulting ether compound may be further purified by an ordinary method such as silica gel column chromatography, distillation and recrystallization.

The used crystalline sulfated Group 4 metal oxide may be recovered and subjected to regeneration treatments such as calcination, and then reused. When the calcination temperature used for preparation of the crystalline sulfated Group 4 metal oxide lies within the above-specified range of from 550 to 750°C, the used crystalline sulfated Group 4

metal oxide may be reused while maintaining its high catalytic activity without being subjected to any regeneration treatments.

EXAMPLES

In the following Examples and Comparative Examples, the terms "%" and "ppm" represent "% by mass" and "ppm by mass", respectively.

(1) The conversion rates of the hydroxyl group-containing compound and the epoxy compound after completion of the reaction may be analyzed and determined using the following gas chromatographic (GC) apparatus and analytic conditions.

<Gas Chromatographic Apparatus and Analytic Conditions>

GC apparatus: "HP 6850 Series" available from Hewlett Packard Co.
Column: "HP-5" available from J & W Corp., (inner diameter: 0.25 mm; length: 30 m; membrane thickness: 0.25 μm)
Carrier gas: He; 1.0 mL/min
Injection condition: 270°C; split ratio: 1/50
Detection condition: FID method; 300°C
Column temperature condition: held at 50°C for 2 min → raised at 10°C/min → held at 300°C for 5 min

(2) The value of $I_{total}$ of the Group 4 metal oxide used in the following Examples, etc., indicating an index of a crystallinity thereof was calculated from a diffraction intensity of a (111) crystal lattice plane and a diffraction intensity of a (-111) crystal lattice plane as observed in powder X-ray diffraction crystal diffraction spectra which were obtained under the following measuring conditions using the following sample preparation method and measuring procedures.

(Measuring Conditions)

Measuring apparatus: "Rigaku RINT 2500VC X-RAY diffractometer" available from Rigaku Corp.
X-ray source: Cu/Kα-radiation
Tube voltage: 40 kV; Tube current: 120 mA
Measuring range: 2θ = 10 to 80°
Sample to be measured: A sample was prepared by compressing pellets having an area of 320 $mm^2$ and a thickness of 1 mm according to the following sample preparation method.
X-ray scanning speed: 10°/min

(Preparation of Sample for Measurement of Powder X-Ray Crystal Diffraction, and Measuring Procedures)

When the sample to be measured has a large particle size, the number of crystals which contribute to X-ray diffraction analysis is lessened, so that a reproducibility of the analysis tends to be deteriorated. Therefore, the sample was fully pulverized and powdered into about 10 μm in diameter using a agate mortar, etc.

When the thus obtained powdered sample was filled in a sampling plate, the sampling plate (an aluminum plate, a glass plate, etc.) was placed on a flat glass plate having a size of 10 cm square and a thickness of about 5 mm in order to reduce an error of a diffraction angle, and the sample was uniformly and evenly filled in a sample filling hole or groove of the sampling plate such that the surface of the sample filled was brought into flush alignment with the surface of the sampling plate.

(3) The contents of a sulfate ion and phosphoric acid in the Group 4 metal oxide were calculated as follows. That is, the contents of sulfur and phosphorus were measured by elemental analysis and converted into amounts of the sulfate ion and phosphoric ion, respectively.

In Examples 1 and 2, the sulfated zirconium oxide available from Wako Pure Chemical Industries, Ltd., was used.

In Examples 3 to 8 and Comparative Examples 5 and 6, there was used the sulfated zirconium oxide available from Daiichi Kigenso Kagaku Kogyo Co., Ltd., which was prepared by the same method as described in JP 11-263621A except for using zirconium hydroxide as a raw material and varying the calcination conditions. The calcination conditions are described in the respective Examples, etc., and Table 2.

EXAMPLE 1

A 200 mL four-necked flask was charged with 62.5 g (0.48 mol) of 2-ethyl hexanol and 1.91 g of sulfated zirconium oxide ($SO_4/ZrO_2$; sulfated zirconia available from Wako Pure Chemical Industries, Ltd.; sulfate ion ($SO_4^{2-}$) content: 9.3%; configuration: powder; X-ray diffraction spectrum: refer to FIG. 1; $I_{total}$: 3550 cps (counts per second)), and the contents of the flask were heated to 100°C while introducing nitrogen thereinto and stirring. Next, 37.0 g (0.40 mol) of epichlorohydrin were dropped into the flask over 1.5 h, and the contents of the flask were continuously stirred as such for 1 h. The resulting reaction mixture was cooled to room temperature and then subjected to filtration to remove the sulfated zirconium oxide therefrom.

As a result of GC analysis, it was confirmed that the conversion rate of epichlorohydrin as the raw material was 100%; the yield of β-2-ethylhexyl chlorohydrin ether produced (β-isomer) was 90.4% and the yield of α-2-ethylhexyl chlorohydrin ether produced (α-isomer) was 2.7% (total yield: 93.1%); and an excessive addition by-product formed by further adding epichlorohydrin to the above 2-ethylhexyl chlorohydrin ether was produced in an amount of 6.6%. The results are shown in Table 1.

EXAMPLE 2

A 200 mL four-necked flask was charged with 53.8 g (0.41 mol) of 2-ethyl hexanol and 1.90 g of the sulfated zirconium oxide used in Example 1, and the contents of the flask were heated to 150°C while introducing nitrogen thereinto and stirring. Next, 37.0 g (0.20 mol) of 2-ethylhexyl glycidyl ether were dropped into the flask over 3.5 h, and the contents of the flask were continuously stirred as such for 1 h. The resulting reaction mixture was cooled to room temperature and then subjected to filtration to remove the sulfated zirconium oxide therefrom.

As a result of GC analysis, it was confirmed that the conversion rate of 2-ethylhexyl glycidyl ether as the raw material was 100%; the yield of 1,3-di-2-ethylhexyl glycerol produced was 83.2% and the yield of 1,2-di-2-ethylhexyl glycerol produced was 2.1% (total yield: 85.3%); and an excessive addition by-product formed by further adding 2-ethylhexyl glycidyl ether to the above 1,2-di-2-ethylhexyl glycerol was produced in an amount of 1.3%. The results are shown in Table 1.

EXAMPLE 3

A 3000 mL four-necked flask was charged with 1426.0 g (7.66 mol) of lauryl alcohol ("Kalcol 2098" available from Kao Corp.) and 23.14 g of sulfated zirconium oxide (available from Daiichi Kigenso Kagaku Kogyo Co., Ltd.; calcined at 600°C (±15°C) for 3 h; sulfate ion ($SO_4^{2-}$) content: 4.1%; configuration: powder; $I_{total}$: 4700 cps (counts per second)), and the contents of the flask were heated to 100°C while introducing nitrogen thereinto and stirring. Next, 463.9 g (5.01 mol) of epichlorohydrin were dropped into the flask over 4 h, and the contents of the flask were continuously stirred as such for 1 h. The resulting reaction mixture was cooled to room temperature and then subjected to filtration to recover the sulfated zirconium oxide therefrom.

As a result of GC analysis, it was confirmed that the conversion rate of epichlorohydrin as the raw material was 100%; and the yield of β-lauryl chlorohydrin ether produced (β-isomer) was 92.0% and the yield of α-lauryl chlorohydrin ether produced (α-isomer) was 2.8% (total yield: 94.8%).

COMPARATIVE EXAMPLE 1

A 1000 mL four-necked flask was charged with 500.7 g (3.85 mol) of 2-ethyl hexanol, 3.40 g (0.016 mol) of aluminum triisopropoxide ($Al(OiPr)_3$; available from Kawaken Fine Chemicals Co., Ltd.) as a catalyst and 2.45 g (0.024 mol) of 98% sulfuric acid, and the contents of the flask were heated to 90°C while introducing nitrogen thereinto and stirring. Next, 296.5 g (3.21 mol) of epichlorohydrin were dropped into the flask over 3 h, and the contents of the flask were continuously stirred as such for 3 h.

As a result of GC analysis, it was confirmed that the conversion rate of epichlorohydrin as the raw material was 100%; the yield of β-2-ethylhexyl chlorohydrin ether produced (β-isomer) was 87.9% and the yield of α-2-ethylhexyl chlorohydrin ether produced (α-isomer) was 1.4% (total yield: 89.3%); and an excessive addition by-product was produced in an amount of 10.2%.

After completion of the reaction, the resulting reaction solution was cooled to 50°C, and 19.23 g (0.048 mol) of

a 10% sodium hydroxide aqueous solution was accurately weighed and added to the reaction solution, followed by stirring the reaction solution for 30 min. Thereafter, the thus obtained reaction solution was neutralized with sodium hydroxide to precipitate an aluminum compound. In this procedure, when the amount of sodium hydroxide added was deficient relative to sulfuric acid, the aluminum compound failed to be completely precipitated, whereas when the amount of sodium hydroxide added was excessive relative to sulfuric acid, the yield of the aimed compound was lowered owing to the reaction between the surplus alkali and the halohydrin ether. Therefore, a stoichiometric amount of sodium hydroxide was added to sulfuric acid added to conduct a strict neutralization. The obtained reaction mixture was cooled to room temperature and then subjected to filtration to remove the aluminum compound therefrom. The results are shown in Table 1.

COMPARATIVE EXAMPLE 2

A 200 mL four-necked flask was charged with 62.6 g (0.48 mol) of 2-ethyl hexanol and 1.89 g "KYOWAAD 700SL" (available from Kyowa Chemical Industry Co., Ltd.; $Al_2O_3/SiO_2$; aluminum oxide: 10.5% and silicon oxide: 60.2%), and the contents of the flask were heated to 100°C while introducing nitrogen thereinto and stirring. Next, 37.0 g (0.40 mol) of epichlorohydrin were dropped into the flask over 1.5 h, and the contents of the flask were continuously stirred as such for 3 h.

As a result of GC analysis, it was confirmed that the conversion rate of epichlorohydrin as the raw material was 46%; the yield of β-2-ethylhexyl chlorohydrin ether produced (β-isomer) was 28.0% and the yield of α-2-ethylhexyl chlorohydrin ether produced (α-isomer) was 1.1% (total yield: 29.1%); and an excessive addition by-product was produced in an amount of 1.6%.

After completion of the reaction, the resulting reaction mixture was cooled to room temperature and then subjected to filtration to remove the catalyst used therefrom. The results are shown in Table 1.

COMPARATIVE EXAMPLE 3

A 200 mL four-necked flask was charged with 1.84 g of phosphoric acid-supporting zirconium oxide (available from Daiichi Kigenso Kagaku Kogyo Co., Ltd.; phosphoric ion ($PO_4^{3-}$) content: 3.6%; configuration: powder; $I_{total}$: 2560 cps (counts per second)) and 62.6 g (0.48 mol) of 2-ethyl hexanol, and the contents of the flask were heated to 100°C while introducing nitrogen thereinto and stirring. Next, 36.8 g (0.40 mol) of epichlorohydrin were dropped into the flask over 1.5 h, and the contents of the flask were continuously stirred as such for 3 h.

As a result of GC analysis, it was confirmed that the conversion rate of epichlorohydrin as the raw material was 42%; the yield of β-2-ethylhexyl chlorohydrin ether produced (β-isomer) was 38.4% and the yield of α-2-ethylhexyl chlorohydrin ether produced (α-isomer) was 0.9% (total yield: 39.3%); and an excessive addition by-product was produced in an amount of 1.5%.

After completion of the reaction, the resulting reaction mixture was cooled to room temperature and then subjected to filtration to remove the catalyst used therefrom. The results are shown in Table 1.

COMPARATIVE EXAMPLE 4

A 200 mL four-necked flask was charged with 62.7 g (0.48 mol) of 2-ethyl hexanol and 11.03 g of the phosphoric acid-supporting zirconium oxide used in Comparative Example 3, and the contents of the flask were heated to 100°C while introducing nitrogen thereinto and stirring. Next, 37.1 g (0.40 mol) of epichlorohydrin were dropped into the flask over 1.5 h, and the contents of the flask were continuously stirred as such for 3 h.

As a result of GC analysis, it was confirmed that the conversion rate of epichlorohydrin as the raw material was 100%; the yield of β-2-ethylhexyl chlorohydrin ether produced (β-isomer) was 84.5% and the yield of α-2-ethylhexyl chlorohydrin ether produced (α-isomer) was 1.7% (total yield: 86.2%); and an excessive addition by-product was produced in an amount of 13.7%.

After completion of the reaction, the resulting reaction mixture was cooled to room temperature and then subjected to filtration to remove the catalyst used therefrom. The results are shown in Table 1.

COMPARATIVE EXAMPLE 5

A 200 mL four-necked flask was charged with 62.6 g (0.48 mol) of 2-ethyl hexanol and 1.84 g of sulfated zirconium oxide (available from Daiichi Kigenso Kagaku Kogyo Co., Ltd.; calcined at 400°C (±15°C) for 3 h; sulfate ion ($SO_4^{2-}$) content: 7.8%; configuration: powder; X-ray diffraction spectrum: refer to FIG. 1; $I_{total}$: 1400 cps (counts per second)), and the contents of the flask were heated to 100°C while introducing nitrogen thereinto and stirring. Next, 37.0 g (0.40 mol) of epichlorohydrin were dropped into the flask over 1.5 h, and the contents of the flask were continuously

stirred as such for 1 h. After completion of the reaction, the resulting reaction mixture was cooled to room temperature and then subjected to filtration to remove the sulfated zirconium oxide therefrom.

As a result of GC analysis, it was confirmed that the conversion rate of epichlorohydrin as the raw material was 56.6%; the yield of β-2-ethylhexyl chlorohydrin ether produced (β-isomer) was 50.7% and the yield of α-2-ethylhexyl chlorohydrin ether produced (α-isomer) was 2.0% (total yield: 52.7%); and an excessive addition by-product formed by further adding epichlorohydrin to the above 2-ethylhexyl chlorohydrin ether was produced in an amount of 0.6%. The results are shown in Table 1.

COMPARATIVE EXAMPLE 6

A 200 mL four-necked flask was charged with 53.3 g (0.41 mol) of 2-ethyl hexanol and 1.89 g of the sulfated zirconium oxide used in Comparative Example 5, and the contents of the flask were heated to 150°C while introducing nitrogen thereinto and stirring. Next, 37.8 g (0.20 mol) of 2-ethylhexyl glycidyl ether were dropped into the flask over 3.5 h, and the contents of the flask were continuously stirred as such for 1 h. After completion of the reaction, the resulting reaction mixture was cooled to room temperature and then subjected to filtration to remove the sulfated zirconium oxide therefrom.

As a result of GC analysis, it was confirmed that the conversion rate of 2-ethylhexyl glycidyl ether as the raw material was 54.0%; the yield of 1,3-di-2-ethylhexyl glycerol produced was 39.4% and the yield of 1,2-di-2-ethylhexyl glycerol produced was 5.4% (total yield: 44.8%); and an excessive addition by-product formed by further adding 2-ethylhexyl glycidyl ether to the above 1,2-di-2-ethylhexyl glycerol was produced in an amount of 0.4%. The results are shown in Table 1.

TABLE 1

| | Catalyst | | | Amount of hydroxyl group-containing compound charged (times by mass) | Time of dropping → aging (h) | Reaction temperature (°C) | Conversion rate of epoxy compound (%) | Yield of ether compound (%)*2 | Method for removal of catalyst |
|---|---|---|---|---|---|---|---|---|---|
| | Kind | $I_{total}$ (cps) | Amount used*1 (%) | | | | | | |
| Example 1 | $SO_4/ZrO_2$ | 3550 | 5 | 1.2 | 1.5 → 1 | 100 | 100 | 93.1 | Filtration |
| Example 2 | $SO_4/ZrO_2$ | 3550 | 5 | 2.0 | 3.5 → 1 | 150 | 100 | 85.3 | Filtration |
| Example 3 | $SO_4/ZrO_2$ | 4700 | 5 | 1.5 | 4 → 1 | 100 | 100 | 94.8 | Filtration |
| Comparative Example 1 | $Al(OiPr)_3$ 98% sulfuric acid | - | 0.5*3 | 1.2 | 3 → 3 | 90 | 100 | 89.3 | Filtration after neutralization |
| Comparative Example 2 | $Al_2O_3/SiO_2$ | - | 5 | 1.2 | 1.5 → 3 | 100 | 46 | 29.1 | Filtration |
| Comparative Example 3 | $PO_4/ZrO_2$ | 2560 | 5 | 1.2 | 1.5 → 3 | 100 | 42 | 39.3 | Filtration |
| Comparative Example 4 | $PO_4/ZrO_2$ | 2560 | 30 | 1.2 | 1.5 → 3 | 100 | 100 | 86.2 | Filtration |
| Comparative Example 5 | $SO_4/ZrO_2$ | 1400 | 5 | 1.2 | 1.5 → 1 | 100 | 57 | 52.7 | Filtration |
| Comparative Example 6 | $SO_4/ZrO_2$ | 1400 | 5 | 2.0 | 3.5 → 1 | 150 | 54 | 44.8 | Filtration |

Note
*1: Amount initially used based on the epoxy compound, and when reused, a whole amount of the catalyst recovered was used;
*2: Total yield based on the epoxy compound;
*3: Mol%

From Table 1, it was confirmed that in Comparative Example 1 (in which the aluminum triisopropoxide and sulfuric acid were used), the strict neutralization treatment for removal of the aluminum compound used as the catalyst was needed, and in Comparative Example 2 (in which the aluminum oxide and silicon oxide were used), Comparative Example 3 (in which the phosphoric acid-supporting zirconium oxide was used) and Comparative Examples 5 and 6 (in which the low-crystalline sulfated zirconium oxide was used), the yield of the aimed product was low owing to a low catalytic activity of the catalyst used.

On the other hand, it was confirmed that in Example 1 according to the present invention (in which the yield of the β-isomer was 90.4%), the sulfated zirconium oxide used therein was able to be readily removed without need of the neutralization treatment as compared to Comparative Example 1, and the selectivity to the aimed product in Example 1 was excellent even as compared to Comparative Example 4 (yield of the β-isomer: 84.5%) in which the amount of the phosphoric acid-supporting zirconia added in Comparative Example 3 was increased to enhance the yield of the aimed product.

In each of the following Examples 4 to 8, the catalyst was recovered after used in the reaction, and then used again for the reaction without subjected to any regeneration treatment to study and evaluate a recycling property thereof.

EXAMPLE 4

A 200 mL four-necked flask was charged with 42.8 g (0.32 mol) of 2-ethyl hexanol and 1.24 g of sulfated zirconium oxide (available from Daiichi Kigenso Kagaku Kogyo Co., Ltd.; calcined at 700°C (±15°C) for 3 h; sulfate ion ($SO_4^{2-}$) content: 3.5%; configuration: powder; $I_{total}$: 5200 cps (counts per second)), and the contents of the flask were heated to 90°C while introducing nitrogen thereinto and stirring. Next, 25.1 g (0.27 mol) of epichlorohydrin were dropped into the flask over 1.5 h, and the contents of the flask were continuously stirred as such for 1 h. After completion of the reaction, the resulting reaction mixture was cooled to room temperature and then subjected to filtration to recover the sulfated zirconium oxide therefrom.

As a result of GC analysis, it was confirmed that the conversion rate of epichlorohydrin as the raw material was 100%; the yield of β-2-ethylhexyl chlorohydrin ether produced (β-isomer) was 90.4% and the yield of α-2-ethylhexyl chlorohydrin ether produced (α-isomer) was 2.7% (total yield: 93.1%); and an excessive addition by-product formed by further adding epichlorohydrin to the above 2-ethylhexyl chlorohydrin ether was produced in an amount of 6.5%.

Further, 43.1 g (0.33 mol) of 2-ethyl hexanol and a whole amount of the hydrous catalyst recovered (1.98 g) were charged into a 200 mL four-necked flask, and the contents of the flask were heated to 90°C while introducing nitrogen thereinto and stirring. Next, 25.3 g (0.27 mol) of epichlorohydrin were dropped into the flask over 1.5 h, and the contents of the flask were continuously stirred as such for 1 h. After completion of the reaction, the resulting reaction mixture was cooled to room temperature and then subjected to filtration to remove the sulfated zirconium oxide therefrom.

As a result of GC analysis, it was confirmed that the conversion rate of epichlorohydrin as the raw material was 100%; the yield of β-2-ethylhexyl chlorohydrin ether produced (β-isomer) was 89.1% and the yield of α-2-ethylhexyl chlorohydrin ether produced (α-isomer) was 2.3% (total yield: 91.4%); and an excessive addition by-product formed by further adding epichlorohydrin to the above 2-ethylhexyl chlorohydrin ether was produced in an amount of 5.9%. The results are shown in Table 2.

EXAMPLE 5

A 200 mL four-necked flask was charged with 43.3 g (0.33 mol) of 2-ethyl hexanol and 1.23 g of sulfated zirconium oxide (available from Daiichi Kigenso Kagaku Kogyo Co., Ltd.; calcined at 600°C (±15°C) for 3 h; sulfate ion ($SO_4^{2-}$) content: 4.1%; configuration: powder; $I_{total}$: 4700 cps (counts per second)), and the contents of the flask were heated to 90°C while introducing nitrogen thereinto and stirring. Next, 25.1 g (0.27 mol) of epichlorohydrin were dropped into the flask over 1.5 h, and the contents of the flask were continuously stirred as such for 1 h. After completion of the reaction, the resulting reaction mixture was cooled to room temperature and then subjected to filtration to recover the sulfated zirconium oxide therefrom.

As a result of GC analysis, it was confirmed that the conversion rate of epichlorohydrin as the raw material was 100%; the yield of β-2-ethylhexyl chlorohydrin ether produced (β-isomer) was 89.4% and the yield of α-2-ethylhexyl chlorohydrin ether produced (α-isomer) was 3.4% (total yield: 92.8%); and an excessive addition by-product formed by further adding epichlorohydrin to the above 2-ethylhexyl chlorohydrin ether was produced in an amount of 6.6%.

Further, 43.5 g (0.33 mol) of 2-ethyl hexanol and a whole amount of the hydrous catalyst recovered (2.66 g) were charged into a 200 mL four-necked flask, and the contents of the flask were heated to 90°C while introducing nitrogen thereinto and stirring. Next, 25.6 g (0.28 mol) of epichlorohydrin were dropped into the flask over 1.5 h, and the contents of the flask were continuously stirred as such for 1 h. After completion of the reaction, the resulting reaction mixture was cooled to room temperature and then subjected to filtration to remove the sulfated zirconium oxide therefrom.

As a result of GC analysis, it was confirmed that the conversion rate of epichlorohydrin as the raw material was

100%; the yield of β-2-ethylhexyl chlorohydrin ether produced (β-isomer) was 88.7% and the yield of α-2-ethylhexyl chlorohydrin ether produced (a-isomer) was 2.3% (total yield: 91.0%); and an excessive addition by-product formed by further adding epichlorohydrin to the above 2-ethylhexyl chlorohydrin ether was produced in an amount of 6.4%. The results are shown in Table 2.

EXAMPLE 6

A 200 mL four-necked flask was charged with 42.9 g (0.32 mol) of 2-ethyl hexanol and 1.25 g of sulfated zirconium oxide (available from Daiichi Kigenso Kagaku Kogyo Co., Ltd.; calcined at 500°C (±15°C) for 3 h; sulfate ion ($SO_4^{2-}$) content: 3.8%; configuration: powder; $I_{total}$: 4000 cps (counts per second)), and the contents of the flask were heated to 90°C while introducing nitrogen thereinto and stirring. Next, 25.7 g (0.28 mol) of epichlorohydrin were dropped into the flask over 1.5 h, and the contents of the flask were continuously stirred as such for 1 h. After completion of the reaction, the resulting reaction mixture was cooled to room temperature and then subjected to filtration to recover the sulfated zirconium oxide therefrom.

As a result of GC analysis, it was confirmed that the conversion rate of epichlorohydrin as the raw material was 100%; the yield of β-2-ethylhexyl chlorohydrin ether produced (β-isomer) was 90.9% and the yield of α-2-ethylhexyl chlorohydrin ether produced (a-isomer) was 3.1% (total yield: 94.0%); and an excessive addition by-product formed by further adding epichlorohydrin to the above 2-ethylhexyl chlorohydrin ether was produced in an amount of 5.9%.

Further, 45.3 g (0.34 mol) of 2-ethyl hexanol and a whole amount of the hydrous catalyst recovered (1.68 g) were charged into a 200 mL four-necked flask, and the contents of the flask were heated to 90°C while introducing nitrogen thereinto and stirring. Next, 24.8 g (0.27 mol) of epichlorohydrin were dropped into the flask over 1.5 h, and the contents of the flask were continuously stirred as such for 5 h. After completion of the reaction, the resulting reaction mixture was cooled to room temperature and then subjected to filtration to remove the sulfated zirconium oxide therefrom.

As a result of GC analysis, it was confirmed that the conversion rate of epichlorohydrin as the raw material was 23.1%; the yield of β-2-ethylhexyl chlorohydrin ether produced (β-isomer) was 21.9% and the yield of α-2-ethylhexyl chlorohydrin ether produced (α-isomer) was 0.7% (total yield: 22.6%); and an excessive addition by-product formed by further adding epichlorohydrin to the above 2-ethylhexyl chlorohydrin ether was produced in an amount of 0.5%. The results are shown in Table 2.

EXAMPLE 7

A 200 mL four-necked flask was charged with 42.7 g (0.32 mol) of 2-ethyl hexanol and 1.25 g of sulfated zirconium oxide (available from Daiichi Kigenso Kagaku Kogyo Co., Ltd.; calcined at 450°C (±15°C) for 3 h; sulfate ion ($SO_4^{2-}$) content: 3.8%; configuration: powder; $I_{total}$: 3950 cps (counts per second)), and the contents of the flask were heated to 90°C while introducing nitrogen thereinto and stirring. Next, 25.4 g (0.27 mol) of epichlorohydrin were dropped into the flask over 1.5 h, and the contents of the flask were continuously stirred as such for 1 h. After completion of the reaction, the resulting reaction mixture was cooled to room temperature and then subjected to filtration to recover the sulfated zirconium oxide therefrom.

As a result of GC analysis, it was confirmed that the conversion rate of epichlorohydrin as the raw material was 100%; the yield of β-2-ethylhexyl chlorohydrin ether produced (β-isomer) was 89.1% and the yield of α-2-ethylhexyl chlorohydrin ether produced (α-isomer) was 3.0% (total yield: 92.1%); and an excessive addition by-product formed by further adding epichlorohydrin to the above 2-ethylhexyl chlorohydrin ether was produced in an amount of 6.9%.

Further, 44.7 g (0.34 mol) of 2-ethyl hexanol and a whole amount of the hydrous catalyst recovered (1.80 g) were charged into a 200 mL four-necked flask, and the contents of the flask were heated to 90°C while introducing nitrogen thereinto and stirring. Next, 25.7 g (0.28 mol) of epichlorohydrin were dropped into the flask over 1.5 h, and the contents of the flask were continuously stirred as such for 5 h. After completion of the reaction, the resulting reaction mixture was cooled to room temperature and then subjected to filtration to remove the sulfated zirconium oxide therefrom.

As a result of GC analysis, it was confirmed that the conversion rate of epichlorohydrin as the raw material was 38.4%; the yield of β-2-ethylhexyl chlorohydrin ether produced (β-isomer) was 36.3% and the yield of α-2-ethylhexyl chlorohydrin ether produced (α-isomer) was 1.1% (total yield: 37.4%); and an excessive addition by-product formed by further adding epichlorohydrin to the above 2-ethylhexyl chlorohydrin ether was produced in an amount of 1.0%. The results are shown in Table 2.

EXAMPLE 8

A 200 mL four-necked flask was charged with 62.6 g (0.48 mol) of 2-ethyl hexanol and 1.84 g of sulfated zirconium oxide (available from Daiichi Kigenso Kagaku Kogyo Co., Ltd.; calcined at 400°C (±15°C) for 3 h; sulfate ion ($SO_4^{2-}$) content: 3.7%; configuration: powder; $I_{total}$: 3970 cps (counts per second)), and the contents of the flask were

heated to 90°C while introducing nitrogen thereinto and stirring. Next, 37.1 g (0.40 mol) of epichlorohydrin were dropped into the flask over 1.5 h, and the contents of the flask were continuously stirred as such for 1 h. After completion of the reaction, the resulting reaction mixture was cooled to room temperature and then subjected to filtration to recover the sulfated zirconium oxide therefrom.

As a result of GC analysis, it was confirmed that the conversion rate of epichlorohydrin as the raw material was 100%; the yield of β-2-ethylhexyl chlorohydrin ether produced (β-isomer) was 91.5% and the yield of α-2-ethylhexyl chlorohydrin ether produced (α-isomer) was 2.6% (total yield: 94.1%); and an excessive addition by-product formed by further adding epichlorohydrin to the above 2-ethylhexyl chlorohydrin ether was produced in an amount of 5.9%.

Further, 62.0 g (0.48 mol) of 2-ethyl hexanol and a whole amount of the hydrous catalyst recovered (2.16 g) were charged into a 200 mL four-necked flask, and the contents of the flask were heated to 90°C while introducing nitrogen thereinto and stirring. Next, 37.1 g (0.40 mol) of epichlorohydrin were dropped into the flask over 1.5 h, and the contents of the flask were continuously stirred as such for 5 h. After completion of the reaction, the resulting reaction mixture was cooled to room temperature and then subjected to filtration to remove the sulfated zirconium oxide therefrom.

As a result of GC analysis, it was confirmed that the conversion rate of epichlorohydrin as the raw material was 78.4%; the yield of β-2-ethylhexyl chlorohydrin ether produced (β-isomer) was 73.3% and the yield of α-2-ethylhexyl chlorohydrin ether produced (α-isomer) was 2.1% (total yield: 75.4%); and an excessive addition by-product formed by further adding epichlorohydrin to the above 2-ethylhexyl chlorohydrin ether was produced in an amount of 2.8%. The results are shown in Table 2.

TABLE 2

| | Catalyst | | | | | | Amount of hydroxyl group-containing compound charged (time by mass) | Time of dropping → aging (h) | Conversion rate of epoxy compound (%) | Yield of ether compound (%)*2 |
|---|---|---|---|---|---|---|---|---|---|---|
| | Kind | Calcination upon preparation of catalyst | | $I_{total}$ (cps) | Amount used (%)*1 | Frequency of use | | | | |
| | | Temperature (°C) | Time (h) | | | | | | | |
| Example 4 | $SO_4/ZrO_2$ | 700±15 | 3 | 5200 | 5 | 1 | 1.2 | 1.5→1 | 100 | 93.1 |
| | | | | | | 2 | 1.2 | 1.5→1 | 100 | 91.4 |
| Example 5 | $SO_4/ZrO_2$ | 600±15 | 3 | 4700 | 5 | 1 | 1.2 | 1.5→1 | 100 | 92.8 |
| | | | | | | 2 | 1.2 | 1.5→1 | 100 | 91.0 |
| Example 6 | $SO_4/ZrO_3$ | 500±15 | 3 | 4000 | 5 | 1 | 1.2 | 1.5→1 | 100 | 94.0 |
| | | | | | | 2 | 1.2 | 1.5→5 | 23.1 | 22.6 |
| Example 7 | $SO_4/ZrO_4$ | 450±15 | 3 | 3950 | 5 | 1 | 1.2 | 1.5→1 | 100 | 92.1 |
| | | | | | | 2 | 1.2 | 1.5→5 | 38.4 | 37.4 |
| Example 8 | $SO_4/ZrO_5$ | 400±15 | 3 | 3970 | 5 | 1 | 1.2 | 1.5→1 | 100 | 94.1 |
| | | | | | | 2 | 1.2 | 1.5→5 | 78.4 | 75.4 |

Note
*1: Amount initially used based on the epoxy compound, and when reused, a whole amount of the catalyst recovered was used;
*2: Total yield based on the epoxy compound

From Table 2, it was confirmed that the crystalline sulfated zirconium oxide used as the catalyst upon the production of the ether compound exhibited a high catalytic activity and a high reaction selectivity irrespective of the calcination temperature used for producing the catalyst.

In addition, from the comparison between Examples 4 and 5 and Examples 6 to 8, it was confirmed that when the calcination temperature used upon the production of the catalyst was in the range of from 550 to 750°C, the resulting catalyst exhibited, even when reused, almost the same high catalytic activity and high selectivity as those upon the initial use, and the catalyst also had a high durability and a high recycling property.

INDUSTRIAL APPLICABILITY

In the process of the present invention, it is possible to produce the aimed ether compound for a short period of time with an extremely high yield. Also, after completion of the reaction, the catalyst used therein can be readily removed.

The ether compound obtained by the process of the present invention can be effectively used with various configurations in the extensive applications such as nonionic surfactants. For example, the ether compound may be used in the form of a compound per se, an aqueous solution, an alcohol solution, a dispersion, an emulsion, a hydrous gel, an oily gel, or a mixture or an impregnated or penetrated product with a solid substance such as waxes in various industrial applications such as cosmetics, perfumes, agricultural chemicals and drugs for the purposes of emulsification, solubilization, dispersion, cleaning, foaming, defoaming, penetration, antibacterial effect, etc.

## Claims

1. A process for producing an ether compound, comprising the step of reacting a hydroxyl group-containing compound with an epoxy compound in the presence of an oxide of a metal of Group 4 of the Periodic Table on which a sulfate ion is supported, wherein

   the hydroxyl group-containing compound is represented by the following general formula (1):

   $$R^1\text{-}(OA^1)_n\text{-}(OA^2)_m\text{-}OH \qquad (1)$$

   wherein $R^1$ is a hydrocarbon group having 1 to 36 carbon atoms; $A^1$ is an alkanediyl group having 2 to 4 carbon atoms; $A^2$ is a hydroxyl group-containing alkanediyl group having 2 to 4 carbon atoms; and n and m represent average polymerization degrees of the $OA^1$ group and the $OA^2$ group, respectively, in which n is a number of from 0 to 20 and m is a number of from 0 to 2,
   the epoxy compound is an $\alpha$-epihalohydrin and/or a 1,2-epoxy compound represented by the following general formula (2):

   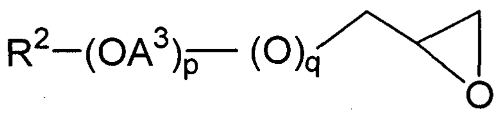
   (2)

   wherein $R^2$ is a hydrocarbon group having 1 to 36 carbon atoms; $A^3$ is an alkanediyl group having 2 to 4 carbon atoms; p is a number of from 0 to 20; and q is a number of 0 or 1, and
   the metal of Group 4 of the Periodic Table is zirconium,
   **characterized in that**
   the sum of the diffraction intensities $I_{total} = (I_{28.3} + I_{30.3} + I_{31.5})$ of the metal oxide is 2000 cps (counts per second) or larger as measured by subjecting the metal oxide to powder X-ray diffraction analysis,
   wherein $I_{28.3}$ represents the diffraction intensity of the (-111) monoclinic lattice plane of the zirconia at a diffraction angle $2\theta = 28.3°$, $I_{30.3}$ represents the diffraction intensity of the (111) tetragonal lattice plane of the zirconia at a diffraction angle $2\theta = 30.3°$, and $I_{31.5}$ represents the diffraction intensity of the (111) monoclinic lattice plane of the zirconia at a diffraction angle $2\theta = 31.5°$,
   wherein the value of $I_{total}$ is obtained under the following measuring conditions and procedure:

   (i) measuring conditions:

   measuring apparatus: "Rigaku RINT 2500VC X-RAY diffractometer" available from Rigaku Corp.,
   X-ray source: Cu/K$\alpha$-radiation,

tube voltage: 40 kV;
tube current: 120 mA,
measuring range: $2\theta$ = 10 to 80°,
sample to be measured: the sample is prepared by compressing to a pellet having an area of 320 $mm^2$ and a thickness of 1 mm in a sample filling hole or groove of the sampling plate according to the following sample preparation procedure,
X-ray scanning speed: 10°/min,

(ii) procedure:

the sample to be measured is fully pulverized and powdered into 10 μm in diameter using an agate mortar, the sampling plate is placed on a flat glass plate having a size of 10 cm square and a thickness of 5 mm, and the sample is uniformly and evenly filled in a sample filling hole or groove of the sampling plate such that the surface of the sample is brought into flush alignment with the surface of the sampling plate.

**2.** The process for producing an ether compound according to claim 1, wherein the 1,2-epoxy compound represented by the general formula (2) is an alkyl glycidyl ether containing an alkyl group having 1 to 36 carbon atoms.

**3.** The process for producing an ether compound according to claim 2, wherein the 1,2-epoxy compound represented by the general formula (2) is 2-ethylhexyl glycidyl ether.

**4.** The process for producing an ether compound according to any one of claims 1 to 3, wherein a content of the sulfate ion in the oxide of the metal of Group 4 of the Periodic Table on which the sulfate ion is supported is from 0.1 to 30% by mass.

**5.** The process for producing an ether compound according to any one of claims 1 to 4, wherein the oxide of the metal of Group 4 of the Periodic Table on which the sulfate ion is supported is produced by calcining a raw metal compound at a temperature of from 550 to 750°C.

**6.** The process for producing an ether compound according to any one of claims 1 to 5, wherein the oxide of the metal of Group 4 of the Periodic Table on which the sulfate ion is supported is used in an amount of from 0.1 to 20% by mass on the basis of the epoxy compound.

**Patentansprüche**

**1.** Verfahren zur Herstellung einer Etherverbindung, umfassend den Schritt des Umsetzens einer Hydroxylgruppen enthaltenden Verbindung mit einer Epoxidverbindung in Gegenwart eines Oxids eines Metalls der Gruppe 4 des Periodensystems, worauf ein Sulfation gelagert ist, wobei
die Hydroxylgruppen enthaltende Verbindung durch die nachstehende allgemeine Formel (1) dargestellt ist:

$$\mathbf{R^1\text{-}(OA^1)_n\text{-}(OA^2)_m\text{-}OH} \qquad (1)$$

wobei $R^1$ eine Kohlenwasserstoffgruppe mit 1 bis 36 Kohlenstoffatomen ist; $A^1$ eine Alkandiylgruppe mit 2 bis 4 Kohlenstoffatomen ist; $A^2$ eine Hydroxylgruppen enthaltende Alkandiylgruppe mit 2 bis 4 Kohlenstoffatomen ist; und n und m durchschnittliche Polymerisierungsgrade der $OA^1$-Gruppe bzw. der $OA^2$-Gruppe darstellen, wobei n eine Zahl von 0 bis 20 und m eine Zahl von 0 bis 2 ist,
wobei die Epoxidverbindung ein α-Epihalogenhydrin und/oder eine 1,2-Epoxidverbindung ist, die durch die nachstehende allgemeine Formel (2) dargestellt ist:

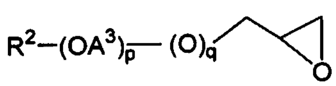

**(2)**

wobei $R^2$ eine Kohlenwasserstoffgruppe mit 1 bis 36 Kohlenstoffatomen ist; $A^3$ eine Alkandiylgruppe mit 2 bis 4 Kohlenstoffatomen ist; p eine Zahl von 0 bis 20 ist; und q eine Zahl von 0 oder 1 ist, und
das Metall der Gruppe 4 des Periodensystems Zirkon ist,

**dadurch gekennzeichnet, dass**
die Summe der Beugungsintensitäten $I_{gesamt} = (I_{28,3} + I_{30,3} + I_{31,5})$ des Metalloxids laut Messung durch Unterziehen des Metalloxids einer Röntgen-Pulverdiffraktionsanalyse 2000 cps (Zählschritte pro Sekunde) oder mehr beträgt, wobei $I_{28,3}$ die Beugungsintensität der monoklinen (-111)-Gitterebene des Zirkondioxids bei einem Beugungswinkel $2\theta = 28,3°$ darstellt, $I_{30,3}$ die Beugungsintensität der tetragonalen (111)-Gitterebene des Zirkondioxids bei einem Beugungswinkel $2\theta = 30,3°$ darstellt und $I_{31,5}$ die Beugungsintensität der monoklinen (111)-Gitterebene des Zirkondioxids bei einem Beugungswinkel $2\theta = 31,5°$ darstellt,
wobei der Wert von $I_{gesamt}$ unter den nachstehenden Messbedingungen und mit dem nachstehenden Verfahrensablauf erhalten wird:

(i). Messbedingungen:

Messgerät: "Rigaku-RINT-2500VC-Röntgen-Diffraktometer", erhältlich von Rigaku Corp.,
Röntgenstrahlenquelle: Cu/K$\alpha$-Strahlung,
Röhrenspannung: 40 kV;
Röhrenstrom: 120 mA,
Messbereich: $2\theta$ = 10 bis 80°,
zu messende Probe: die Probe wird durch Verdichtung zu einem Pellet mit einer Fläche von 320 $mm^2$ und einer Dicke von 1 mm in einem Probenfüllloch oder einer Nut der Probennahmeplatte gemäß dem nachstehenden Probenaufbereitungsverfahrensablauf aufbereitet,
Röntgen-Abtastgeschwindigkeit: 10°/min,

(ii). Verfahrensablauf:

die zu messende Probe wird mithilfe eines Achatmörsers vollständig pulverisiert und auf einen Durchmesser von 10 $\mu$m zerrieben, die Probennahmeplatte wird auf eine flache Glasplatte mit einer Größe von 10 $cm^2$ und einer Dicke von 5 mm platziert, und die Probe wird gleichmäßig und einheitlich in ein Probenfüllloch oder eine Nut der Probennahmeplatte gefüllt, sodass die Oberfläche der Probe in eine bündige Ausrichtung mit der Oberfläche der Probennahmeplatte gebracht wird.

2. Verfahren zur Herstellung einer Etherverbindung nach Anspruch 1, wobei die durch die allgemeine Formel (2) dargestellte 1,2-Epoxidverbindung ein Alkylglycidylether ist, das eine Alkylgruppe mit 1 bis 36 Kohlenstoffatomen enthält.

3. Verfahren zur Herstellung einer Etherverbindung nach Anspruch 2, wobei die durch die allgemeine Formel (2) dargestellte 1,2-Epoxidverbindung 2-Ethylhexylglycidylether ist.

4. Verfahren zur Herstellung einer Etherverbindung nach einem der Ansprüche 1 bis 3, wobei ein Gehalt des Sulfations in dem Oxid des Metalls der Gruppe 4 des Periodensystems, auf dem das Sulfation gelagert ist, 0,1 bis 30 Massen-% beträgt.

5. Verfahren zur Herstellung einer Etherverbindung nach einem der Ansprüche 1 bis 4, wobei das Oxid des Metalls der Gruppe 4 des Periodensystems, auf dem das Sulfation gelagert ist, durch Kalzinieren einer Rohmetallverbindung bei einer Temperatur von 550 bis 750 °C hergestellt wird.

6. Verfahren zur Herstellung einer Etherverbindung nach einem der Ansprüche 1 bis 5, wobei das Oxid des Metalls der Gruppe 4 des Periodensystems, auf dem das Sulfation gelagert ist, in einer Menge von 0,1 bis 20 Massen-%, bezogen auf die Epoxidverbindung, verwendet wird.

**Revendications**

1. Procédé de production d'un composé éther, comprenant l'étape de mise en réaction d'un composé contenant un groupe hydroxyle avec un composé époxy en présence d'un oxyde d'un métal du groupe 4 du tableau périodique sur lequel un ion sulfate est supporté, dans lequel
le composé contenant un groupe hydroxyle est représenté par la formule générale (1) suivante :

$$R^1\text{-}(OA^1)_n\text{-}(OA^2)_m\text{-}OH \qquad (1)$$

dans lequel $R^1$ est un groupe hydrocarboné ayant de 1 à 36 atomes de carbone ; $A^1$ est un groupe alcanediyle ayant de 2 à 4 atomes de carbone ; $A^2$ est un groupe alcanediyle contenant un groupe hydroxyle ayant de 2 à 4 atomes de carbone ; et n et m représentent des degrés de polymérisation moyens du groupe $OA^1$ et du groupe $OA^2$, respectivement, dans lequel n est un nombre de 0 à 20 et m est un nombre de 0 à 2,
le composé époxy est une $\alpha$-épihalohydrine et/ou un composé 1,2-époxy représenté par la formule générale (2) suivante :

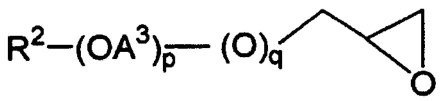 (2)

dans lequel $R^2$ est un groupe hydrocarboné ayant de 1 à 36 atomes de carbone ; $A^3$ est un groupe alcanediyle ayant de 2 à 4 atomes de carbone ; p est un nombre de 0 à 20 ; et q est un nombre de 0 ou 1, et
le métal du groupe 4 du tableau périodique est le zirconium,
**caractérisé en ce que**
la somme des intensités de diffraction $I_{totale} = (I_{28,3} + I_{30,3} + I_{31,5})$ de l'oxyde de métal est de 2 000 cps (coups par seconde) ou plus telle que mesurée par soumission de l'oxyde de métal à une analyse par diffraction des rayons X sur poudre,
dans lequel $I_{28,3}$ représente l'intensité de diffraction du plan de réseau monoclinique (-111) de la zircone à un angle de diffraction $2\theta$ = 28,3°, $I_{30,3}$ représente l'intensité de diffraction du plan de réseau tétragonal (111) de la zircone à un angle de diffraction $2\theta$ = 30,3°, et $I_{31,5}$ représente l'intensité de diffraction du plan de réseau monoclinique (111) de la zircone à un angle de diffraction $2\theta$ = 31,5°,
dans lequel la valeur $I_{totale}$ est obtenue dans les conditions de mesure suivantes et conformément à la procédure suivante :

(i) conditions de mesure :

appareil de mesure : « diffractomètre Rigaku RINT 2500VC X-RAY » disponible auprès de Rigaku Corp.
source de rayons X : rayonnement Cu/K$\alpha$
tension du tube : 40 kV ;
courant du tube : 120 mA,
plage de mesure : $2\theta$ = 10 à 80°,
échantillon à mesurer : l'échantillon est préparé par compression en une pastille ayant une aire de 320 $mm^2$ et une épaisseur de 1 mm dans un trou ou une gorge de remplissage d'échantillon de la plaque d'échantillonnage conformément à la procédure de préparation d'échantillon suivante,
vitesse de balayage par rayons X : 10°/min

(ii) procédure :

l'échantillon à mesurer est totalement pulvérisé et réduit en poudre avec un diamètre de 10 $\mu$m à l'aide d'un mortier d'agate, la plaque d'échantillonnage est placée sur une plaque en verre plate ayant une taille de 10 $cm^2$ et une épaisseur de 5 mm, et l'échantillon est rempli de façon uniforme et homogène dans un trou ou une gorge de remplissage d'échantillon de la plaque d'échantillonnage de telle sorte que la surface de l'échantillon se trouve en alignement par affleurement avec la surface de la plaque d'échantillonnage.

**2.** Procédé de production d'un composé éther selon la revendication 1, dans lequel le composé 1,2-époxy représenté par la formule générale (2) est un alkyl glycidyl éther contenant un groupe alkyle ayant de 1 à 36 atomes de carbone.

**3.** Procédé de production d'un composé éther selon la revendication 2, dans lequel le composé 1,2-époxy représenté par la formule générale (2) est le 2-éthylhexyl glycidyl éther.

**4.** Procédé de production d'un composé éther selon l'une quelconque des revendications 1 à 3, dans lequel une teneur en l'ion sulfate dans l'oxyde du métal du groupe 4 du tableau périodique sur lequel l'ion sulfate est supporté est de 0,1 à 30 % en masse.

**5.** Procédé de production d'un composé éther selon l'une quelconque des revendications 1 à 4, dans lequel l'oxyde

du métal du groupe 4 du tableau périodique sur lequel l'ion sulfate est supporté est produit par calcination d'un composé de métal brut à une température de 550 à 750°C.

**6.** Procédé de production d'un composé éther selon l'une quelconque des revendications 1 à 5, dans lequel l'oxyde du métal du groupe 4 du tableau périodique sur lequel l'ion sulfate est supporté est utilisé en une quantité de 0,1 à 20 % en masse sur la base du composé époxy.

FIG. 1

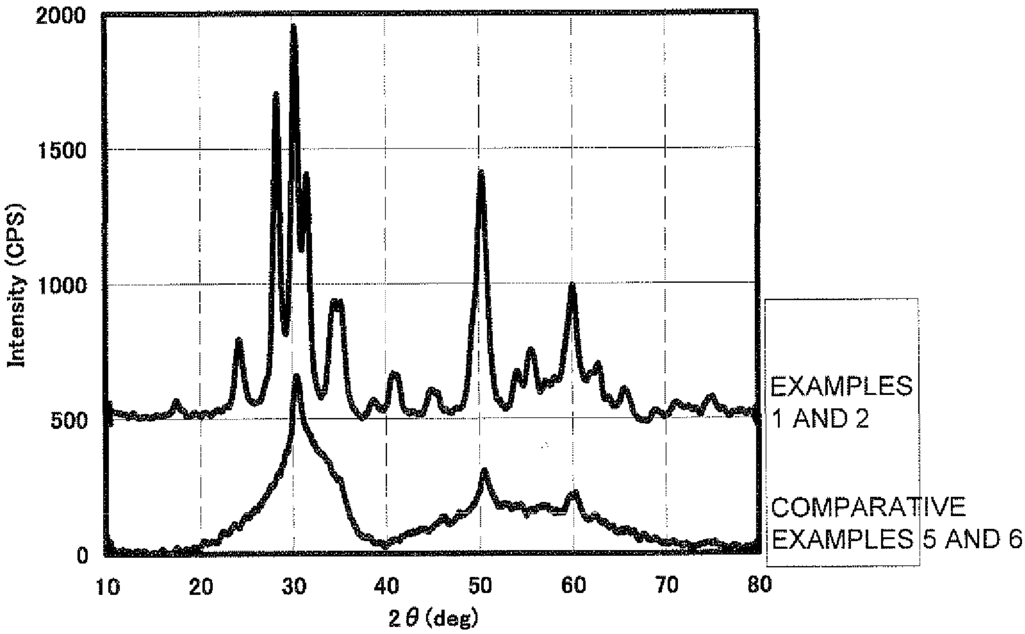
2000
1500
1000
500
0
Intensity (CPS)
10
20
30
40
50
60
70
80
2θ (deg)
EXAMPLES 1 AND 2
COMPARATIVE EXAMPLES 5 AND 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10036307 A
- JP 47020606 B
- JP 54014910 A
- JP 7133269 A
- US 4727199 A
- US 4873017 A
- US 5025094 A
- JP 11263621 A


### Non-patent literature cited in the description


- *PETEROTECH,* 1996, vol. 19 (9), 733